# EUROPEAN PATENT SPECIFICATION

(11) **EP 3 747 424 B1**
(45) Date of publication and mention of the grant of the patent: **26.03.2025**
(21) Application number: 19748241.7
(22) Date of filing: 30.01.2019
(51) Int. Cl.: A61Q 19/00, A61Q 19/02, A61K 8/24, A61K 8/41, A61K 8/44, A61K 8/55, A61K 8/60

(54) **METHOD FOR SUPPRESSING DISCOLORATION OF EXTERNAL COMPOSITION CONTAINING ADENOSINE MONOPHOSPHATE AND TRANEXAMIC ACID**
VERFAHREN ZUR UNTERDRÜCKUNG DER VERFÄRBUNG EINER EXTERNEN ZUSAMMENSETZUNG MIT ADENOSINMONOPHOSPHAT UND TRANEXAMSÄURE
PROCÉDÉ DE SUPPRESSION DE LA DÉCOLORATION D'UNE COMPOSITION EXTERNE CONTENANT DU MONOPHOSPHATE D'ADÉNOSINE ET DE L'ACIDE TRANEXAMIQUE

(30) Priority: 31.01.2018 JP 2018015486
(43) Date of publication of application: 09.12.2020
(73) Proprietor: Otsuka Pharmaceutical Co., Ltd., Tokyo 101-8535 (JP)
(72) Inventor: OGIHARA, Miyoko, Osaka-shi, Osaka 540-0021 (JP); SUMA, Momoko, Osaka-shi, Osaka 540-0021 (JP); MIYAWAKI, Shiori, Osaka-shi, Osaka 540-0021 (JP)
(74) Representative: Hoffmann Eitle
(86) International application number: PCT/JP2019/003058
(87) International publication number: WO 2019/151276

(56) References cited:
- EP-A1- 0 685 229
- CN-A- 104 434 766
- JP-A- 2003 081 840
- JP-A- 2003 206 224
- JP-A- 2014 062 077
- US-A1- 2006 183 708
- DATABASE GNPD [online] MINTEL; 29 May 2001 (2001-05-29), ANONYMOUS: "Luminizing Night Essence Lotion", XP055887309, retrieved from https://www.gnpd.com/sinatra/recordpage/10086323/ Database accession no. 10086323
- DATABASE GNPD [online] MINTEL; 1 October 2015 (2015-10-01), ANONYMOUS: "Skin Whitening Cream", XP055887316, retrieved from https://www.gnpd.com/sinatra/recordpage/3514209/ Database accession no. 3514209
- DATABASE GNPD [online] MINTEL; 22 March 2011 (2011-03-22), ANONYMOUS: "Whitening Enliver", XP055887319, retrieved from https://www.gnpd.com/sinatra/recordpage/1535818/ Database accession no. 1535818

## Description

### Technical Field

The present application relates to a method for suppressing discoloration of a composition for external use comprising an adenosine monophosphate and/or a salt thereof and tranexamic acid and/or a salt thereof, and to such composition suppressed in discoloration.

### Background Art

An adenosine phosphate is known to have a moisturizing effect and a whitening effect, and cosmetics comprising an adenosine phosphate have already been put on the market.

Tranexamic acid has an anti-plasmin effect and is blended in cosmetics as an active ingredient for improving rough skin and whitening, and such cosmetics have already been on the market.

Patent Document 1 discloses a composition for preventing or improving pigmentation comprising an adenosine monophosphate and tranexamic acid.

DATABASE GNPD [Online], MINTEL; 29 May 2001 (2001-05-29), anonymous: "Luminizing Night Essence Lotion", Database accession no. 10086323, discloses a cosmetic composition for external use comprising disodium adenosine triphosphate, tranexamic acid and trisodium EDTA. This lotion further comprises PEG-6 and PEG-20.

DATABASE GNPD [Online], MINTEL; 1 October 2015 (2015-10-01), anonymous: "Skin Whitening Cream", Database accession no. 3514209, relates to a cosmetic composition for external use comprising adenosine monophosphate, tranexamic acid and disodium EDTA. This cream further comprises bis-ethoxydiglycol cyclohexane 1,4-dicarboxylate.

DATABASE GNPD [Online], MINTEL; 22 March 2011 (2011-03-22), anonymous: "Whitening Enliver", Database accession no. 1535818, exemplifies a cosmetic composition for external use comprising disodium adenosine triphosphate, tranexamic acid and trisodium EDTA, as well as sodium hexametaphosphate. This serum further comprises PEG-6, PEG-20 and silica.

EP 0 685 229 A1 describes a preparation for external application to the skin which comprises disodium adenosine triphosphate and tranexamic acid for prevention of skin roughening and skin improvement. The preparation further contains ginseng extract.

CN 104 434 766 A describes a nursing spray containing cordyceps militaris. The nursing spray contains 22 active ingredients including aloe yeast, hydrolyzed oat protein, a cordyceps sinensis extracting solution, hydrolyzed royal jelly, a purslane extract, a persimmon leaf extract, an orange peel extract, a scutellaria baicalensis root extract, a phellinus linteus extract, a white willow bark extract, a vanilla extract, a Lengxiang rose extract, a mung bean extract, a green tea extract, an illicium verum extract, an astragalus membranaceus extract, a panax ginseng extract, a tricholoma matsutake extract, a rice bran extract, a jasmine extract, a peach kernel extract and a linalool leaf extract.

### Prior Art Document

### Patent Document

Patent Document 1: WO 2006/033343

### Summary of Invention

### Technical Problem

The present inventors tried to develop a composition comprising an adenosine monophosphate and tranexamic acid, and encountered the problem that the composition discolored with time (especially yellowing). An object of the present invention is to provide a method for suppressing discoloration with time in a composition comprising an adenosine monophosphate and/or a salt thereof and tranexamic acid and/or a salt thereof, and to provide a composition in which the discoloration is suppressed.

### Solution to Problem

The present inventors have studied intensively to solve the above problem, and found that specific chelating agents and specific pH adjusters can respectively suppress the discoloration with time of a composition comprising an adenosine monophosphate and/or a salt thereof and tranexamic acid and/or a salt thereof, thereby reaching the present invention. In addition, there has been a problem that such compositions provide a sticky feeling due to an adenosine monophosphate and/or a salt thereof, and the present inventors have found that it is preferable to blend specific ingredient(s) (ingredients for suppressing sticky feeling) in order to suppress the sticky feeling, and further found that some specific ingredient(s) among these ingredients for suppressing sticky feeling do not deteriorate the discoloration with time.

Thus, the present application provides the following:
[1] A composition for external use, comprising:
   Ingredient (A): an adenosine monophosphate and/or a salt thereof; and
   Ingredient (B): tranexamic acid and/or a salt thereof, and further comprising
   Ingredient (C): a chelating agent which is a phosphate compound selected from tripolyphosphoric acid or a salt thereof, metaphosphoric acid or a salt thereof, etidronic acid or a salt thereof, and a mixture thereof; and/or
   Ingredient (D): a pH adjuster which is an organic alkali compound.
[2] A method for producing a composition for external use, comprising mixing Ingredient (A): an adenosine monophosphate and/or a salt thereof; and Ingredient (B) : tranexamic acid and/or a salt thereof, in the presence of Ingredient (C): a chelating agent which is a phosphate compound selected from tripolyphosphoric acid or a salt thereof, metaphosphoric acid or a salt thereof, etidronic acid or a salt thereof, and a mixture thereof; and/or Ingredient (D) : a pH adjuster which is an organic alkali compound.
[3] A method for suppressing discoloration of a composition for external use which comprises Ingredient (A): an adenosine monophosphate and/or a salt thereof; and Ingredient (B): tranexamic acid and/or a salt thereof,
   comprising using Ingredient (C): a chelating agent which is a phosphate compound selected from tripolyphosphoric acid or a salt thereof, metaphosphoric acid or a salt thereof, etidronic acid or a salt thereof, and a mixture thereof; and/or Ingredient (D): a pH adjuster which is an organic alkali compound.

### Effect of The Invention

The present invention provides a composition for external use, comprising an adenosine monophosphate and/or a salt thereof, and tranexamic acid and/or a salt thereof, in which the discoloration with time is suppressed.

### Description of Embodiment

The present application relates to a composition for external use, comprising
Ingredient (A): an adenosine monophosphate and/or a salt thereof; and
Ingredient (B): tranexamic acid and/or a salt thereof; and further comprising
Ingredient (C): a chelating agent which is a phosphate compound selected from tripolyphosphoric acid or a salt thereof, metaphosphoric acid or a salt thereof, etidronic acid or a salt thereof, and a mixture thereof; and/or
Ingredient (D): a pH adjuster which is an organic alkali compound.

The composition comprising an adenosine monophosphate and/or a salt thereof and tranexamic acid and/or a salt thereof may be inhibited from discoloration with time by compounding Ingredient (C) and/or Ingredient (D).

In the present application, a salt of adenosine monophosphate is not particularly limited as long as it can be formulated in a cosmetic, a drug or quasi-drug for external use. Examples of a salt of adenosine monophosphate include specifically alkali metal salts such as sodium salts, potassium salts; alkaline earth metal salts such as calcium salts, magnesium salts, barium salts; basic amino acid salts such as arginine, lysine; ammonium salts such as ammonium salts, tricyclohexylammonium salts; alkanolamine salts such as monoethanolamine salts, diethanolamine salts, triethanolamine salts, monoisopropanolamine salts, diisopropanolamine salts, triisopropanolamine, and a single kind of the salt of adenosine monophosphate may be used, or any combination of two or more kinds of the salt of adenosine monophosphate may be used.

The amount of Ingredient (A) included in the composition of the present application may vary depending on the use or form of the composition for external use, but may be optionally selected from a range of, for example, usually 0.001 to 10% by weight based on the total weight of the composition for external use. Preferably from 0.01% by weight to 10% by weight, more preferably from 0.1% by weight to 7% by weight, more preferably from 0.5% by weight to 5% by weight, still more preferably from 0.7% by weight to 3% by weight, and even more preferably from 1% by weight to 2% by weight are exemplified.

Further examples of the lower limit of the amount range of Ingredient (A) included in the composition for external use of the application include 0.001% by weight, 0.01% by weight, 0.1% by weight, 0.5% by weight, 0.7% by weight, and 1% by weight, and examples of the upper limit include 1% by weight, 2% by weight, 3% by weight, 5% by weight, 7% by weight, and 10% by weight, and preferred examples of the range may be shown by a combination of the lower limit and the upper limit.

In the present application, a salt of tranexamic acid is not particularly limited as long as it can be formulated in a cosmetic, a drug or quasi-drug for external use. Examples of a salt of tranexamic acid include alkali metal salts, alkaline earth metal salts, basic amino acid salts, ammonium salts, alkanolamine salts, as mentioned above for an adenosine monophosphate, and a single kind of salt of tranexamic acid may be used, or any combination of two or more kinds of salt of tranexamic acid may be used.

The amount of Ingredient (B) included in the composition of the present application may vary depending on the use or form of the composition for external use, but may be optionally selected from, for example, a range of usually 0.001 to 10% by weight based on the total weight of the composition for external use. Preferably from 0.01% by weight to 10% by weight, more preferably from 0.1% by weight to 7% by weight, more preferably from 0.5% by weight to 5% by weight, still more preferably from 0.7% by weight to 3% by weight, and even more preferably from 1% by weight to 2% by weight are exemplified.

Further, examples of the lower limit of the amount range of Ingredient (B) included in the composition for external use of the application include 0.001% by weight, 0.01% by weight, 0.1% by weight, 0.5% by weight, 0.7% by weight, and 1% by weight, and examples of the upper limit include 1% by weight, 2% by weight, 3% by weight, 5% by weight, 7% by weight, and 10% by weight, and preferred examples of the range may be indicated by a combination of the lower limit and the upper limit.

Examples of the chelating agent for Ingredient (C) include tripolyphosphoric acid or a salt thereof, metaphosphoric acid or a salt thereof, and etidronic acid or a salt thereof, and a single kind of the chelating agent may be used, or any combination of two or more kinds of the chelating agent may be used. In the composition of the present application, chelating agent(s) other than Ingredient (C) may be used to the extent that discoloration with time is not problematic.

Examples of a salt of tripolyphosphoric acid, a salt of metaphosphoric acid, a salt of etidronic acid include alkali metal salts, alkaline earth metal salts, basic amino acid salts, ammonium salts, alkanolamine salts, as mentioned above for an adenosine monophosphate. A single kind of the salt of these may be used, or any combination of two or more kinds of the salt of these may be used.

Preferred examples of a salt of tripolyphosphoric acid include sodium tripolyphosphate and potassium tripolyphosphate.

Preferred examples of a salt of metaphosphoric acid include sodium metaphosphate and potassium metaphosphate.

Preferred examples of a salt of etidronic acid include trisodium etidronate, tetrapotassium etidronate, and tetrasodium etidronate.

In the composition for external use of the present application, the amount of Ingredient (C) is not particularly limited as long as the discoloration with time of the composition is suppressed, but it is preferable that Ingredient (C) is comprised in a weight ratio of 0.00001 to 0.5 (more preferably 0.0001 to 0.2, still more preferably 0.0002 to 0.1) based on the sum of Ingredient (A) and Ingredient (B), that is, (Ingredient (A) + Ingredient (B)): Ingredient (C) = 1: 0.00001 to 0.5 (more preferably 1: 0.0001 to 0.2, still more preferably 1: 0.0002 to 0.1). Further, examples of the lower limit value of the weight ratio range of Ingredient (C) when the total weight of Ingredient (A) and Ingredient (B) comprised in the composition for external use of the present application is 1 include 0.00001, 0.0001, 0.0002, 0.0005, 0.001, 0.01, 0.05, examples of the upper limit value include 0.05, 0.1, 0.2, 0.5, and preferred examples of the range may be indicated by a combination of the lower limit value and the upper limit value.

The amount of Ingredient (C) that may be comprised in the composition of the present application may be optionally selected, for example, from a range of usually 0.0001 to 2% by weight based on the total weight of the composition for external use. Preferably 0.0002 to 1.5% by weight, more preferably 0.0005 to 1% by weight, and more preferably 0.001 to 0.5% by weight are exemplified.

Further examples of the lower limit of the amount range of Ingredient (C) that may be included in the composition for external use of the application include 0.0001% by weight, 0.0002% by weight, 0.0005% by weight, 0.001% by weight, 0.01% by weight, 0.1% by weight, and 0.2% by weight, and examples of the upper limit include 0.2% by weight, 0.5% by weight, 1% by weight, and 2% by weight, and preferred examples of the range may be indicated by a combination of the lower limit and the upper limit.

Examples of a pH adjuster which is an organic alkali compound of Ingredient (D) include aminohydroxymethylpropanediol, aminomethylpropanol, aminomethylpropanediol, arginine, and triethanolamine, and a single kind of the pH adjuster may be used, or any combination of two or more kinds of the pH adjuster may be used. Preferred examples of a pH adjuster which is an organic alkali compound include aminohydroxymethylpropanediol, aminomethylpropanediol, and arginine.

In order to suppress discoloration with time, it is preferable to use the pH adjuster of Ingredient (D) for adjusting the pH of the composition for external use of the present application. The preferred pH of the composition for external use of the present application may vary depending on the use of the composition, but may be pH 5.5 to 7.5. In order to adjust pH of the composition for external use of the present application, a pH adjuster other than Ingredient (D) may be used to the extent that discoloration with time is not problematic.

In the composition for external use of the present application, the amount of Ingredient (D) is not particularly limited as long as the discoloration with time of the composition is suppressed, and may vary depending on the amount of Ingredient (A), the kind and amount of optional ingredient(s), and the preferable pH of the composition, but for example, Ingredient (D) may be comprised in a weight ratio of 0.0001 to 4 (more preferably 0.001 to 2, still more preferably 0.005 to 1) based on the Ingredient (A), that is, Ingredient (A): Ingredient (D) = 1: 0.0001 to 4 (more preferably 1: 0.001 to 2, still more preferably 1: 0.005 to 1).

Further, examples of the lower limit of the weight ratio range of Ingredient (C) when the weight of Ingredient (A) comprised in the composition for external use of the present application is 1 include 0.0001, 0.001, 0.01, and 0.1, examples of the upper limit include 0.1, 0.5, 1, 2, 3, and 4, and preferred examples of the range may be indicated by a combination of the lower limit and the upper limit.

The composition suppressed in sticky feeling and suppressed in discoloration with time can be provided by compounding Ingredient (E): bis-ethoxydiglycol cyclohexane dicarboxylate, diethoxyethyl succinate, highly polymerized polyethylene glycol (for example, those with a freezing point of 40 °C or higher), highly polymerized silicone (for example, those with a kinematic viscosity of 6000 mm²/s or more), pullulan, polyvinylpyrrolidone, an organic fine particle (for example, silica, poly(methyl methacrylate), talc, and urethane), biosaccharide gum-1 or a mixture thereof.

In order to provide better suppression of sticky feeling in the composition for external use of the present application, it is preferable that Ingredient (A) and Ingredient (E) are comprised in a weight ratio of Ingredient (A): Ingredient (E) = 1: 0.01 to 30 (more preferably 1: 0.02 to 25, still more preferably 1: 0.1 to 20) .

Further, examples of the lower limit of the range of the weight ratio of Ingredient (E) when the weight of Ingredient (A) comprised in the composition for external use of the present application is 1 include 0.01, 0.03, 0.05, 0.1, 0.5, 0.7, 1, examples of the upper limit include 1.5, 2, 3, 5, 10, 20, 25, 30, and preferred examples of the range may be indicated by a combination of the lower limit and the upper limit.

The amount of Ingredient (E) included in the composition of the present application may vary depending on the kind of Ingredient (E) to be used and the use or form of the composition for external use, but may be optionally selected from a range of, for example, usually 0.001 to 10% by weight based on the total weight of the composition for external use. Preferably from 0.01% by weight to 10% by weight, more preferably from 0.1% by weight to 7% by weight, more preferably from 0.5% by weight to 5% by weight, and even more preferably from 0.7% by weight to 2% by weight are exemplified.

Further, examples of the lower limit of the amount range of Ingredient (E) included in the composition for external use of the present application include 0.001% by weight, 0.01% by weight, 0.1% by weight, 0.5% by weight, 0.7% by weight, and 1% by weight, and examples of the upper limit include 1% by weight, 2% by weight, 3% by weight, 5% by weight, 7% by weight, and 10% by weight, and preferred examples of the range may be indicated by a combination of the lower limit and the upper limit.

Each ingredient that may be compounded into the composition of the present application may be in the form of a hydrate.

The composition for external use of the present application may be prepared in various forms by combining pharmaceutically or cosmetically acceptable base(s) or carrier(s) in addition to the above ingredients. For pharmaceutically or cosmetically acceptable base(s) and carrier(s), conventionally known one(s) can be used. The composition of the present invention may comprise, if required, a wide variety of known ingredients used for externally-applied compositions suitable for the skin or mucous membranes, such as cosmetics or externally-applied medical/quasi-medical drugs. Examples of such ingredients include surfactants, colorants (dyes and pigments), flavors, preservatives, bactericides (antibacterials), thickeners, antioxidants, sequestering agents, cooling agents, deodorizers, humectants, UV absorbers, UV dispersants, vitamins, plant extracts, skin astringents, antiinflammatory agents (antiphlogistic agents), whitening agents, cell activators, vasodilators, blood circulation accelerators, skin function accelerators.

For example, the composition for external use of the present application may optionally further comprise water, ethanol, glycerin, BG, 1,2-pentanediol, menthol, POE hydrogenated castor oil, phenoxyethanol, flavors, and a mixture thereof.

The composition for external use of the present invention may be used as a composition for external use to be applied or sprayed on the skin. Specifically, the composition of the present invention may be used as an external preparation (skin preparation) for cosmetics, external medicines or external quasi-medicines.

The form of the composition for external use of the present invention is not particularly limited as long as it can be applied to the skin, and examples thereof include a paste, a mousse, a gel, a liquid, a milky liquid, a suspension liquid, a cream, an ointment, a solid, a sheet, an aerosol, a spray, and a liniment. Especially when using as a cosmetic, lotion; emulsions such as emollient emulsion, milky lotion, nourishing emulsion, and cleansing emulsion; cream such as emollient cream, massage cream, cleansing cream, and makeup cream; a lip balm.

### Example

The present invention is explained in further detail with reference to Formulation Examples and Test Examples. However, the scope of the invention is not limited to these Examples.

### <Test Example 1> Chelating agent for suppressing discoloration - I

The ingredients as shown in the following table were mixed and adjusted to pH 5.5 to 7.5 with potassium hydroxide to provide the comparative example and each of the formulation examples, which were filled in a closed glass container and stored at 60 °C for 2 weeks.

After the storage, a visual check was conducted to evaluate discoloration as below,
⊚: Not discolored;
∘: Almost not discolored;
△: Slightly discolored;
X: Markedly discolored.

The results are shown in the table below.

**[Table 1-1]**

| | Compar ative exampl e | Formul ation exampl e 1 | Formul ation exampl e 2 | Formul ation exampl e 3 | Formul ation exampl e 4 | Formul ation exampl e 5 | Formul ation exampl e 6 |
|---|---|---|---|---|---|---|---|
| Water | Balance | Balance | Balance | Balance | Balance | Balance | Balance |
| Adenosine monophosphate | 2 | 2 | 2 | 2 | 2 | 2 | 2 |
| Tranexamic acid | 2 | 2 | 2 | 2 | 2 | 2 | 2 |
| 1,2-Pentanediol | 1 | 1 | 1 | 1 | 1 | 1 | 1 |
| Sodium tripolyphosphate | | 0.001 | 0.005 | 0.01 | 0.05 | 0.1 | 0.2 |
| Result | X | ○ | ⊚ | ⊚ | ⊚ | ⊚ | ⊚ |

**[Table 1-2]**

| | Formu latio n examp le 7 | Formu latio n examp le 8 | Formu latio n examp le 9 | Formu latio n examp le 10 | Formu latio n examp le 11 | Formu latio n examp le 12 | Formu latio n examp le 13* | Formu latio n examp le 14* |
|---|---|---|---|---|---|---|---|---|
| Water | Balanc e | Balanc e | Balanc e | Balanc e | Balanc e | Balanc e | Balanc e | Balanc e |
| Adenosine monophosphate | 2 | 2 | 2 | 2 | 2 | 2 | 2 | 2 |
| Tranexamic acid | 2 | 2 | 2 | 2 | 2 | 2 | 2 | 2 |
| 1,2-Pentanediol | 1 | 1 | 1 | 1 | 1 | 1 | 1 | 1 |
| Sodium metaphosphate | 0.01 | 0.05 | 0.1 | 0.2 | | | | |
| Etidronic acid | | | | | 0.06 | | | |
| Phytic acid | | | | | | 0.1 | | |
| EDTA·2Na·2H₂O | | | | | | | 0.2 | |
| EDTA·4Na·4H₂O | | | | | | | | 0.2 |
| Result | ⊚ | ⊚ | ⊚ | ⊚ | ⊚ | ○ | ○ | ○ |

| | | | | | | | | |
|---|---|---|---|---|---|---|---|---|
| *Reference Examples | | | | | | | | |

### <Test Example 2> Chelating agent for suppressing discoloration - II

The ingredients as shown in the following table were mixed and adjusted to pH 5.5 to 7.5 with potassium hydroxide to prepare the comparative example and each of the formulation examples, which were filled in a closed glass container and stored at 50 °C for 6 months.

After the storage, a visual check was conducted to evaluate discoloration as below,
⊚: Not discolored;
∘: Almost not discolored;
△: Slightly discolored;
X: Markedly discolored.

The results are shown in the table below.

"Other ingredients" in the comparative example and each of the formulation examples consists of the same ingredients: humectants (glycerin, butylene glycol, and 1,2-pentanediol), touch improver (bis-ethoxydiglycol cyclohexane dicarboxylate), preservative (phenoxyethanol), solubilizer (POE hardened castor oil), and flavor, and the comparative example and each of the formulation examples comprised the same amount of "Other ingredients".

**[Table 2]**

| | Comparat ive example | Formulat ion example 15 | Formulat ion example 16 | Formulat ion example 17 | Formulat ion example 18* | Formulat ion example 19* |
|---|---|---|---|---|---|---|
| Water | Balance | Balance | Balance | Balance | Balance | Balance |
| Adenosine monophosphate | 1 | 1 | 1 | 1 | 1 | 1 |
| Tranexamic acid | 2 | 2 | 2 | 2 | 2 | 2 |
| Ethanol | 7 | 7 | 7 | 7 | 7 | 7 |
| Other ingredients | Suitable amount | Suitable amount | Suitable amount | Suitable amount | Suitable amount | Suitable amount |
| Sodium tripolyphosphate | | 0.2 | | | | |
| Sodium metaphosphate | | | 0.2 | | | |
| Etidronic acid | | | | 0.2 | | |
| Phytic acid | | | | | 0.2 | |
| EDTA·2Na·2H₂O | | | | | | 0.2 |
| Result | x | ⊚ | ⊚ | ⊚ | ○ | ○ |

| | | | | | | |
|---|---|---|---|---|---|---|
| *Reference Examples | | | | | | |

### <Test Example 3> pH adjuster for suppressing discoloration

The ingredients as shown in the following table were mixed to provide the comparative example and each of the formulation examples (pH of these examples were 5.5 to 7.5), which were filled in a closed glass container and stored at 60 °C for 2 weeks.

After the storage, a visual check was conducted to evaluate discoloration as below,
⊚: Not discolored;
∘: Almost not discolored;
△: Slightly discolored;
X: Markedly discolored.

The results are shown in the table below.

**[Table 3]**

| | Formula tion example 20 | Formula tion example 21 | Formula tion example 22 | Formula tion example 23 | Comparative example | |
|---|---|---|---|---|---|---|
| Water | Balance | Balance | Balance | Balance | Balance | Balance |
| Adenosine monophosphate | 2 | 2 | 2 | 2 | 2 | 2 |
| Tranexamic acid | 2 | 2 | 2 | 2 | 2 | 2 |
| Phenoxyethanol | 0.2 | 0.2 | 0.2 | 0.2 | 0.2 | 0.2 |
| Aminohydroxymethylpr opanediol | 1.1 | | | | | |
| Aminomethylpropanedi ol | | 1.1 | | | | |
| Arginine | | | 1.9 | | | |
| Triethanolamine | | | | 1.6 | | |
| Potassium hydroxide | | | | | 0.6 | |
| Sodium hydroxide | | | | | | 0.3 |
| Result | ⊚ | ⊚ | ⊚ | ○ | x | x |

### <Example 4> Agent for suppressing sticky feeling and not accelerating discoloration

The ingredients as shown in the following table were mixed and adjusted to pH 5.5 to 7.5 with potassium hydroxide to prepare the comparative example and each of the formulation examples. In "Other ingredients", a preservative (phenoxyethanol), a solubilizing agent and a flavor were comprised.

### (Evaluation of sticky feeling)

The comparative example and each of the formulation examples (0.5 g) were applied to the faces of three panelists. The sticky feelings of the formulation examples were evaluated as below.
⊚: Suppressed compared with the comparative example;
∘: Slightly suppressed compared with the comparative example;
x: Not suppressed compared with the comparative example;

The results are shown in Table 4.

### (Evaluation of discoloration)

The comparative example and each of the formulation examples were filled in a closed glass container and stored at 50 °C for 1 week or 40 °C for 6 months.

After the storage, a visual check was conducted to evaluate discoloration as below,
⊚: Not discolored;
○: Almost not discolored;
△: Slightly discolored;
X: Markedly discolored.

The results are shown in Table 4.

**[Table 4-1]**

| % by weight | Compa rativ e examp le | Formu latio n examp le 24 | Formu latio n examp le 25 | Formu latio n examp le 26 | Formu latio n examp le 27 | Formu latio n examp le 28 | Formu latio n examp le 29 | Formu latio n examp le 30 | Formu latio n examp le 31 |
|---|---|---|---|---|---|---|---|---|---|
| Water | Balan ce | Balan ce | Balan ce | Balan ce | Balan ce | Balan ce | Balan ce | Balan ce | Balan ce |
| Adenosine monophosphat e | 2 | 2 | 2 | 2 | 1 | 1 | 1 | 1 | 1 |
| Tranexamic acid | 2 | 2 | 2 | 2 | 2 | 2 | 2 | 2 | 2 |
| Ethanol | 7 | 7 | 7 | 7 | 7 | 7 | 7 | 7 | 7 |
| Sodium tripolyphosp hate | 0.2 | 0.2 | 0.2 | 0.2 | 0.2 | 0.2 | 0.2 | 0.2 | 0.2 |
| Other ingredients | Suita ble amoun t | Suita ble amoun t | Suita ble amoun t | Suita ble amoun t | Suita ble amoun t | Suita ble amoun t | Suita ble amoun t | Suita ble amoun t | Suita ble amoun t |
| Bis-ethoxydiglyc ol cyclohexane dicarboxylat e | | 0.5 | | | | | | | |
| Diethoxyethy 1 succinate | | | 1 | | | | | | |
| Highly polymerized polyethylene glycol | | | | 2 | | | | | |
| Highly polymerized dimethicone | | | | | 0.02 | | | | |
| Pullulan | | | | | | 0.1 | | | |
| Polyvinylpyr rolidone | | | | | | | 0.1 | | |
| Silica | | | | | | | | 2 | |
| poly (methyl methacrylate ) | | | | | | | | | 2 |
| Visual check (50 °C for 1 week) | ⊚ | ⊚ | ⊚ | ⊚ | ⊚ | ⊚ | ⊚ | ⊚ | ⊚ |
| Visual check (40 °C for 6 months) | ○ | ○ | ○ | ○ | ○ | ○ | ○ | ○ | ○ |
| Sticky feeling | x | ⊚ | ⊚ | ⊚ | ⊚ | ⊚ | ⊚ | ⊚ | ⊚ |

**[Table 4-2]**

| % by weight | Formu latio n examp le 32 | Formu latio n examp le 33 | Formu latio n examp le 34 | Formu latio n examp le 35 | Formu latio n examp le 36 | Formu latio n examp le 37 | Formu latio n examp le 38 | Formu latio n examp le 39 | Formu latio n examp le 40 |
|---|---|---|---|---|---|---|---|---|---|
| Water | Balan ce | Balan ce | Balan ce | Balan ce | Balan ce | Balan ce | Balan ce | Balan ce | Balan ce |
| Adenosine monophosphate | 1 | 1 | 2 | 2 | 2 | 1 | 2 | 2 | 1 |
| Tranexamic acid | 2 | 2 | 2 | 2 | 2 | 2 | 2 | 2 | 2 |
| Ethanol | 7 | 7 | 7 | 7 | 7 | 7 | 7 | 7 | 7 |
| Sodium tripolyphospha te | 0.2 | 0.2 | 0.2 | 0.2 | 0.2 | 0.2 | 0.2 | 0.2 | 0.2 |
| Other ingredients | Suita ble amoun t | Suita ble amoun t | Suita ble amoun t | Suita ble amoun t | Suita ble amoun t | Suita ble amoun t | Suita ble amoun t | Suita ble amoun t | Suita ble amoun t |
| Talc | 2 | | | | | | | | |
| Urethane | | 2 | | | | | | | |
| Biosaccharide gum-1 | | | 0.05 | | | | | | |
| PEG/PPG/Polybu tylene glycol - 8/5/3 glycerin | | | | 1 | | | | | |
| Diglycerin | | | | | 3 | | | | |
| Polyglycerin | | | | | | 1 | | | |
| Polyoxypropyle ne diglyceryl ether | | | | | | | 1 | | |
| Polyoxyethylen e methyl glucoside | | | | | | | | 1 | |
| Polyglyceryl-10 Eicosanedioate /Tetradecanedi oate | | | | | | | | | 1.5 |
| Discoloration (50 °C for 1 week) | ⊚ | ⊚ | ⊚ | ○ | △ | △ | △ | △ | △ |
| Discoloration (40 °C for 6 months) | ○ | ○ | ○ | △ | - | - | - | - | - |
| Sticky feeling | ⊚ | ⊚ | ⊚ | ⊚ | ⊚ | ⊚ | ⊚ | ⊚ | ⊚ |

Formulation examples comprising 1% by weight, 1.5% by weight or 2% by weight of bis-ethoxydiglycol cyclohexane dicarboxylate were also tested. The results were the same as the formulation example comprising 0.5% by weight of bis-ethoxydiglycol cyclohexane dicarboxylate shown in Table 4.

Formulation examples comprising 1.5% by weight or 2% by weight of diethoxyethyl succinate were also tested. The results were the same as the formulation example comprising 1% by weight of diethoxyethyl succinate shown in Table 4.

Formulation examples comprising 2.5% by weight, 3% by weight, 3.5% by weight, 4% by weight, 4.5% by weight or 5% by weight of silica were also tested. The results were the same as the formulation example comprising 2% by weight of silica shown in Table 4.

Formulation examples comprising 2.5% by weight, 3% by weight, 3.5% by weight, 4% by weight, 4.5% by weight or 5% by weight of poly(methyl methacrylate) were also tested. The results were the same as the formulation example comprising 2% by weight of poly(methyl methacrylate) shown in Table 4.

Formulation examples comprising 2.5% by weight, 3% by weight, 3.5% by weight, 4% by weight, 4.5% by weight or 5% by weight of talc were also tested. The results were the same as the formulation example comprising 2% by weight of talc shown in Table 4.

Formulation examples comprising 2.5% by weight, 3% by weight, 3.5% by weight, 4% by weight, 4.5% by weight or 5% by weight of urethane were also tested. The results were the same as the formulation example comprising 2% by weight of urethane shown in Table 4.

Formulation examples comprising 1.5% by weight, 2% by weight, 2.5% by weight or 3% by weight of PEG/PPG/Polybutylene glycol - 8/5/3 glycerin were also tested. The results were the same as the formulation example comprising 1% by weight of PEG/PPG/Polybutylene glycol - 8/5/3 glycerin shown in Table 4.

Formulation examples comprising 1.5% by weight, 2% by weight, 2.5% by weight, 3% by weight, 3.5% by weight, 4% by weight, 4.5% by weight, or 5% by weight of polyglycerin were also tested. The results were the same as the formulation example comprising 1% by weight of polyglycerin shown in Table 4.

Formulation examples comprising 1.5% by weight, 2% by weight, 2.5% by weight, 3% by weight, 3.5% by weight, 4% by weight, 4.5% by weight, or 5% by weight of polyoxypropylene diglyceryl ether were also tested. The results were the same as the formulation example comprising 1% by weight of polyoxypropylene diglyceryl ether shown in Table 4.

Formulation examples comprising 2% by weight, 2.5% by weight, 3% by weight, 3.5% by weight, 4% by weight, 4.5% by weight, or 5% by weight of Polyglyceryl-10 Eicosanedioate/Tetradecanedioate were also tested. The results were the same as the formulation example comprising 1.5% by weight of Polyglyceryl-10 Eicosanedioate/Tetradecanedioate shown in Table 4.

### Formulation example 41: Skin lotion

### <Ingredients>% by weight

| | |
|---|---|
| Adenosine phosphate | 1.5 |
| Tranexamic acid | 1.5 |
| Glycerin | 8 |
| Butylene glycol | 4 |
| Hydroxylated lecithin | 0.1 |
| Hyaluronic acid | 0.02 |
| Polyoxyethylene polyoxypropylene decyl tetradecyl ether | 0.2 |
| Etidronic acid | 0.02 |
| Aminomethylpropanediol | Suitable amount |
| Preservative | Suitable amount |
| Flavor | Suitable amount |
| Purified water | Balance |

### <Manufacturing method>

According to a conventional method, the water-soluble ingredients were dissolved in purified water to provide a main phase. The water-insoluble ingredient mixed with the surfactants were added to the main phase to provide the skin lotion.

### Formulation Example 42: White-turbid skin lotion

### <Ingredients> % by weight

| | |
|---|---|
| Adenosine phosphate | 0.5 |
| Tranexamic acid | 3 |
| Glycerin | 1 |
| Dipropylene glycol | 4 |
| Polyethylene glycol 20000 | 1 |
| Ethanol | 7 |
| Polyoxyethylene hydrogenated castor oil | 0.4 |
| Polyoxyethylene glyceryl isostearate | 0.2 |
| Pentaerythrityl tetraethylhexanoate | 0.2 |
| Sodium tripolyphosphate | 0.1 |
| Aminohydroxymethylpropanediol | Suitable amount |
| Preservative | Suitable amount |
| Flavor | Suitable amount |
| Purified water | Balance |

### <Manufacturing method>

According to a conventional method, the water-soluble ingredients were dissolved in purified water to provide an aqueous phase. The water-insoluble ingredients were mixed with the surfactants with heating, and the resulting mixture was gradually added to the aqueous phase to provide the white-turbid lotion.

### Example 43: Beauty essence

### <Ingredients>% by weight

| | |
|---|---|
| Adenosine phosphate | 1 |
| Tranexamic acid | 2 |
| Ascorbic acid glucoside | 2 |
| Glycerin | 8 |
| Sorbitol | 2 |
| Oligohyaluronic acid | 0.1 |
| Xanthan gum | 0.2 |
| Quince seed gum | 0.1 |
| Polyoxyethylene hydrogenated castor oil | 0.3 |
| Etidronic acid | 0.03 |
| Aminomethylpropanol | Suitable amount |
| Preservative | Suitable amount |
| Antioxidant | Suitable amount |
| Flavor | Suitable amount |
| Purified water | Balance |

### <Manufacturing method>

According to a conventional method, the water-soluble ingredients were dissolved in purified water to provide a main phase. The water-insoluble ingredients mixed with the surfactant were added to the main phase to provide the beauty essence.

### Formulation example 44: Beauty essence gel

| | |
|---|---|
| Adenosine phosphate | 1 |
| Tranexamic acid | 1.5 |
| Carboxyvinyl polymer | 0.2 |
| PEG-240/HDI copolymer bis-decyltetradeceth-20 ether | 1 |
| Xanthan gum | 0.2 |
| Polyoxyethylene polyoxypropylene decyl tetradecyl ether | 0.3 |
| Diethoxyethyl succinate | 1.5 |
| Butylene glycol | 5 |
| Glycerin | 3 |
| Sodium tripolyphosphate | 0.1 |
| Aminohydroxymethylpropanediol | Suitable amount |
| Preservative | Suitable amount |
| Antioxidant | Suitable amount |
| Flavor | Suitable amount |
| Purified water | Balance |

### <Manufacturing method>

The carbomer was dispersed in purified water, and then the other water-soluble ingredients were added thereto and dissolved by warming to provide a main phase. The water-insoluble ingredients mixed with the surfactant were added to the main phase. The resulting mixture was mixed uniformly and deaerated to provide the desired beauty essence gel.

### Formulation example 45: Gel emulsion

### <Ingredients>% by weight

| | |
|---|---|
| Adenosine Phosphate | 0.5 |
| Tranexamic Acid | 2 |
| Carboxyvinyl polymer | 0.2 |
| Acrylate/alkyl methacrylate copolymer | 0.5 |
| Glycerin | 6 |
| Dipropylene glycol | 4 |
| Cyclopentasiloxane | 5 |
| Dimethicone | 1 |
| Dimethiconol | 0.2 |
| Aminopropyl dimethicone | 0.1 |
| Liquid isoparaffin | 2 |
| Sodium metaphosphate | 0.4 |
| Arginine | Suitable amount |
| Preservative | Suitable amount |
| Antioxidant | Suitable amount |
| Flavor | Suitable amount |
| Purified water | Balance |

### <Manufacturing method>

The water-soluble polymers and other water-soluble ingredients were dissolved in purified water with warming to provide an aqueous phase. The oil phase, in which the oil-soluble ingredients were dissolved by warming, was dispersed in the aqueous phase and mixed with a disper, and emulsified. After mixing uniformly, the mixture was deaerated to provide the desired gel emulsion.

### Formulation Example 46: Emulsion

### <Ingredient>% by weight

| | |
|---|---|
| Adenosine phosphate | 0.5 |
| Tranexamic acid | 2 |
| Glycerin | 5 |
| Butylene glycol | 3 |
| Polyoxyethylene hydrogenated castor oil | 3 |
| Pentaerythrityl tetraethylhexanoate | 4 |
| Phytosteryl/behenyl/octyldodecyl lauroyl glutamate | 0.5 |
| Xanthan gum | 0.4 |
| EDTA-2Na | 0.3 |
| Aminomethylpropanediol | Suitable amount |
| Preservative | Suitable amount |
| Antioxidant | Suitable amount |
| Flavor | Suitable amount |
| Purified water | Balance |

### <Manufacturing method>

The water-soluble polymer and other water-soluble ingredients were dissolved in purified water with warming to provide an aqueous phase. The oil phase, in which the oil-soluble ingredients were dissolved by warming, was dispersed in aqueous phase and mixed with a disper, and emulsified. After mixing uniformly, the mixture was deaerated to provide the desired emulsion.

### Example 47: Cream

### <Ingredients>% by weight

| | |
|---|---|
| Adenosine phosphate | 1.5 |
| Tranexamic acid | 2 |
| Glycerin | 5 |
| Trehalose | 1 |
| Polyoxyethylene glyceryl monoisostearate | 2 |
| Glyceryl stearate | 3 |
| Sodium stearoyl glutamate | 0.5 |
| Triethylhexanoin | 8 |
| Liquid paraffin | 7 |
| Vaseline | 1 |
| Dimethicone | 0.5 |
| Behenyl alcohol | 3 |
| Stearic acid | 2 |
| Polyvinyl alcohol | 0.5 |
| Hydroxyethyl cellulose | 0.5 |
| Phytic acid | 0.4 |
| Aminohydroxymethylpropanediol | Suitable amount |
| Preservative | Suitable amount |
| Antioxidant | Suitable amount |
| Flavor | Suitable amount |
| Purified water | Balance |

### <Manufacturing method>

The water-soluble polymers and other water-soluble ingredients were dissolved in purified water with warming to provide an aqueous phase. The oil phase, in which the oil-soluble ingredients were dissolved by warming, was dispersed in the aqueous phase and mixed with a disper, and emulsified. After mixing uniformly, the mixture was deaerated to provide the desired cream.

## Claims

1. A composition for external use, comprising:
Ingredient (A) : an adenosine monophosphate and/or a salt thereof; and
Ingredient (B): tranexamic acid and/or a salt thereof, and further comprising
Ingredient (C): a chelating agent which is a phosphate compound selected from tripolyphosphoric acid or a salt thereof, metaphosphoric acid or a salt thereof, etidronic acid or a salt thereof, and a mixture thereof; and/or
Ingredient (D): a pH adjuster which is an organic alkali compound.

2. The composition according to claim 1, wherein Ingredient (D) is selected from aminohydroxymethylpropanediol, aminomethylpropanol, aminomethylpropanediol, arginine, triethanolamine, and a mixture thereof.

3. The composition according to claim 1 or 2, wherein Ingredient (C) is comprised in a weight ratio of (Ingredient (A) + Ingredient (B)): Ingredient (C) = 1: 0.0001 to 0.5.

4. The composition according to any one of claims 1 to 3, which comprises Ingredient (D) and has pH of 5.5 to 7.5.

5. The composition according to any one of claims 1 to 4, wherein Ingredient (D) is comprised in a weight ratio of Ingredient (A): Ingredient (D) = 1: 0.00001 to 4.

6. The composition according to any one of claims 1 to 5, which comprises 0.01 to 10% by weight of Ingredient (A).

7. The composition according to any one of claims 1 to 6, which comprises 0.01 to 10% by weight of Ingredient (B).

8. The composition according to any one of claims 1 to 7, further comprising Ingredient (E): bis-ethoxydiglycol cyclohexane dicarboxylate, diethoxyethyl succinate, highly polymerized polyethylene glycol, highly polymerized silicone, pullulan, polyvinylpyrrolidone, an organic fine particle, biosaccharide gum-1, or a mixture thereof.

9. A method for producing a composition for external use, comprising mixing Ingredient (A): an adenosine monophosphate and/or a salt thereof; and Ingredient (B): tranexamic acid and/or a salt thereof, in the presence of Ingredient (C): a chelating agent which is a phosphate compound selected from tripolyphosphoric acid or a salt thereof, metaphosphoric acid or a salt thereof, etidronic acid or a salt thereof, and a mixture thereof; and/or Ingredient (D): a pH adjuster which is an organic alkali compound.

10. A method for suppressing discoloration of a composition for external use which comprises Ingredient (A): an adenosine monophosphate and/or a salt thereof; and Ingredient (B): tranexamic acid and/or a salt thereof,
comprising using Ingredient (C): a chelating agent which is a phosphate compound selected from tripolyphosphoric acid or a salt thereof, metaphosphoric acid or a salt thereof, etidronic acid or a salt thereof, and a mixture thereof; and/or Ingredient (D): a pH adjuster which is an organic alkali compound.

11. The method according to claim 9, wherein Ingredient (D) is selected from aminohydroxymethylpropanediol, aminomethylpropanol, aminomethylpropanediol, arginine, triethanolamine, and a mixture thereof.

12. The method according to claim 10, wherein Ingredient (D) is selected from aminohydroxymethylpropanediol, aminomethylpropanol, aminomethylpropanediol, arginine, triethanolamine, and a mixture thereof.

## Patentansprüche

1. Zusammensetzung zur äußerlichen Anwendung, umfassend:
Bestandteil (A): ein Adenosinmonophosphat und/oder ein Salz davon; und
Bestandteil (B): Tranexamsäure und/oder ein Salz davon,
und ferner umfassend:
Bestandteil (C): einen Chelatbildner, der eine Phosphatverbindung ist, die aus Tripolyphosphorsäure oder einem Salz davon, Metaphosphorsäure oder einem Salz davon, Etidronsäure oder einem Salz davon und einer Mischung davon ausgewählt ist; und/oder
Bestandteil (D): einen pH-Einsteller, der eine organische Alkaliverbindung ist.

2. Zusammensetzung gemäß Anspruch 1, wobei Bestandteil (D) aus Aminohydroxymethylpropandiol, Aminomethylpropanol, Aminomethylpropandiol, Arginin, Triethanolamin und einer Mischung davon ausgewählt ist.

3. Zusammensetzung gemäß Anspruch 1 oder 2, wobei Bestandteil (C) in einem Gewichtsverhältnis von (Bestandteil (A) + Bestandteil (B)): Bestandteil (C) = 1: 0,0001 bis 0,5 enthalten ist.

4. Zusammensetzung gemäß einem der Ansprüche 1 bis 3, die Bestandteil (D) umfasst und einen pH-Wert von 5,5 bis 7,5 aufweist.

5. Zusammensetzung gemäß einem der Ansprüche 1 bis 4, wobei Bestandteil (D) in einem Gewichtsverhältnis von Bestandteil (A): Bestandteil (D) = 1: 0,00001 bis 4 enthalten ist.

6. Zusammensetzung gemäß einem der Ansprüche 1 bis 5, umfassend 0,01 bis 10 Gew.-% des Bestandteils (A).

7. Zusammensetzung gemäß einem der Ansprüche 1 bis 6, umfassend 0,01 bis 10 Gew.-% des Bestandteils (B).

8. Zusammensetzung gemäß einem der Ansprüche 1 bis 7, ferner umfassend Bestandteil (E): Bis-Ethoxydiglycol-Cyclohexandicarboxylat, Diethoxyethylsuccinat, hochpolymerisiertes Polyethylenglycol, hochpolymerisiertes Silikon, Pullulan, Polyvinylpyrrolidon, einen organischen Feinpartikel, Biosaccharid Gum-1 oder eine Mischung davon.

9. Verfahren zur Herstellung einer Zusammensetzung zur äußerlichen Anwendung, umfassend das Mischen von Bestandteil (A): einem Adenosinmonophosphat und/oder einem Salz davon; und Bestandteil (B): Tranexamsäure und/oder einem Salz davon, in Gegenwart von Bestandteil (C): einem Chelatbildner, der eine Phosphatverbindung ist, die aus Tripolyphosphorsäure oder einem Salz davon, Metaphosphorsäure oder einem Salz davon, Etidronsäure oder einem Salz davon und einer Mischung davon ausgewählt ist; und/oder von Bestandteil (D): einem pH-Einsteller, der eine organische Alkaliverbindung ist.

10. Verfahren zur Unterdrückung der Verfärbung einer Zusammensetzung zur äußerlichen Anwendung, die Bestandteil (A): ein Adenosinmonophosphat und/oder ein Salz davon; und Bestandteil (B): Tranexamsäure und/oder ein Salz davon umfasst,
umfassend die Verwendung von Bestandteil (C): einem Chelatbildner, der eine Phosphatverbindung ist, die aus Tripolyphosphorsäure oder einem Salz davon, Metaphosphorsäure oder einem Salz davon, Etidronsäure oder einem Salz davon und einer Mischung davon ausgewählt ist; und/oder von Bestandteil (D): einem pH-Einsteller, der eine organische Alkaliverbindung ist.

11. Verfahren gemäß Anspruch 9, wobei Bestandteil (D) aus Aminohydroxymethylpropandiol, Aminomethylpropanol, Aminomethylpropandiol, Arginin, Triethanolamin und einer Mischung davon ausgewählt ist.

12. Verfahren gemäß Anspruch 10, wobei Bestandteil (D) aus Aminohydroxymethylpropandiol, Aminomethylpropanol, Aminomethylpropandiol, Arginin, Triethanolamin und einer Mischung davon ausgewählt ist.

## Revendications

1. Composition pour utilisation externe, comprenant :
Ingrédient (A) : un adénosine monophosphate et/ou un sel de celui-ci ; et
Ingrédient (B) : acide tranéxamique et/ou un sel de celui-ci, et comprenant en outre
Ingrédient (C) : un agent chélatant qui est un composé phosphate choisi dans acide tripolyphosphorique ou un sel de celui-ci, acide métaphosphorique ou un sel de celui-ci, acide étidronique ou un sel de celui-ci, et un mélange de ceux-ci ; et/ou
Ingrédient (D) : un ajusteur de pH qui est un composé alcalin organique.

2. Composition selon la revendication 1, dans laquelle l'Ingrédient (D) est choisi dans aminohydroxyméthylpropanediol, aminométhylpropanol, aminométhylpropanediol, arginine, triéthanolamine, et un mélange de ceux-ci.

3. Composition selon la revendication 1 ou 2, dans laquelle l'Ingrédient (C) est compris dans un rapport de poids de (Ingrédient (A) + Ingrédient (B)) : Ingrédient (C) = 1:0,0001 à 0,5.

4. Composition selon l'une quelconque des revendications 1 à 3, qui comprend l'Ingrédient (D) et présente un pH de 5,5 à 7,5.

5. Composition selon l'une quelconque des revendications 1 à 4, dans laquelle l'Ingrédient (D) est compris dans un rapport de poids de Ingrédient (A) : Ingrédient (D) = 1:0,00001 à 4.

6. Composition selon l'une quelconque des revendications 1 à 5, qui comprend 0,01 % en poids à 10 % en poids d'Ingrédient (A).

7. Composition selon l'une quelconque des revendications 1 à 6, qui comprend 0,01 % en poids à 10 % en poids d'Ingrédient (B).

8. Composition selon l'une quelconque des revendications 1 à 7, comprenant en outre l'Ingrédient (E) : bis-éthoxydiglycol cyclohexane dicarboxylate, succinate de diéthoxyéthyle, polyéthylène glycol hautement polymérisé, silicone hautement polymérisé, pullulane, polyvinylpyrrolidone, une particule fine organique, gomme biosaccharide 1, ou un mélange de ceux-ci.

9. Procédé de production d'une composition pour utilisation externe, comprenant le mélange de l'Ingrédient (A) : un adénosine monophosphate et/ou un sel de celui-ci ; et de l'Ingrédient (B) : acide tranéxamique et/ou un sel de celui-ci, en présence de l'Ingrédient (C): un agent chélatant qui est un composé phosphate choisi dans acide tripolyphosphorique ou un sel de celui-ci, acide métaphosphorique ou un sel de celui-ci, acide étidronique ou un sel de celui-ci, et un mélange de ceux-ci ; et/ou de l'Ingrédient (D) : un ajusteur de pH qui est un composé alcalin organique.

10. Procédé de suppression de décoloration de composition pour utilisation externe qui comprend l'Ingrédient (A) : un adénosine monophosphate et/ou un sel de celui-ci ; et l'Ingrédient (B) : acide tranéxamique et/ou un sel de celui-ci,
comprenant l'utilisation de l'Ingrédient (C) : un agent chélatant qui est un composé phosphate choisi dans acide tripolyphosphorique ou un sel de celui-ci, acide métaphosphorique ou un sel de celui-ci, acide étidronique ou un sel de celui-ci, et un mélange de ceux-ci ; et/ou de l'Ingrédient (D) : un ajusteur de pH qui est un composé alcalin organique.

11. Procédé selon la revendication 9, dans lequel l'Ingrédient (D) est choisi dans aminohydroxyméthylpropanediol, aminométhylpropanol, aminométhylpropanediol, arginine, triéthanolamine, et un mélange de ceux-ci.

12. Procédé selon la revendication 10, dans lequel l'Ingrédient (D) est choisi dans aminohydroxyméthylpropanediol, aminométhylpropanol, aminométhylpropanediol, arginine, triéthanolamine, et un mélange de ceux-ci.
